# EUROPEAN PATENT SPECIFICATION

(11) **EP 2 768 324 B1**
(45) Date of publication and mention of the grant of the patent: **01.02.2017**
(21) Application number: 12786858.6
(22) Date of filing: 19.10.2012
(51) Int. Cl.: A61K 35/20, A61P 3/04, A23L 33/19, A23L 33/10

(54) **USE OF WHEY PROTEIN MICELLES FOR ENHANCING ENERGY EXPENDITURE AND SATIETY**
VERWENDUNG VON MOLKEPROTEINMIZELLEN ZUR VERBESSERUNG DES ENERGIEVERBRAUCHS UND DER SAETTIGKEIT
UTILISATION DE MICELLES DE PROTÉINES DE LACTOSÉRUM POUR AMÉLIORER LA DÉPENSE ÉNERGÉTIQUE ET LA SATIÉTÉ

(30) Priority: 21.10.2011 EP 11186146
(43) Date of publication of application: 27.08.2014
(73) Proprietor: Nestec S.A., 1800 Vevey (CH)
(72) Inventor: POUTEAU, Etienne, 7550042 Las Condes Santiago (CL); ACHESON, Kevin John, CH-1037 Etagnières (CH); BOVETTO, Lionel Jean René, F-74500 Larringes (FR); BREUILLE, Denis, CH-1010 Lausanne (CH)
(74) Representative: Chautard, Cécile
(86) International application number: PCT/EP2012/070713
(87) International publication number: WO 2013/057229

(56) References cited:
- WO-A1-2011/112695
- WO-A2-2006/034857
- US-A- 5 882 705
- US-A1- 2011 250 310
- US-B1- 6 767 575
- ACHESON K J ET AL: "Protein choices targeting thermogenesis and metabolism", THE AMERICAN JOURNAL OF CLINICAL NUTRITION, AMERICAN SOCIETY FOR NUTRITION, US, vol. 93, no. 3, 1 January 2011 (2011-01-01), pages 525-534, XP009156726, ISSN: 0002-9165

## Description

The present invention relates to whey protein micelles for use in the treatment and/or prevention of overweight and/or obesity in a subject. The invention relates also to a non-therapeutic use of whey protein micelles to increase satiety and/or postprandial energy expenditure in a subject. A further aspect related to the invention is a food composition to be administered to an overweight or obese subject, or to a subject at risk for becoming overweight or obese.

During the past decades, the prevalence of obesity has increased worldwide to epidemic proportion. Approximately 1 billion of people worldwide are overweight or obese, conditions that increase mortality, mobility and economical costs. Overweight and obesity are classically defined based on the percentage of body fat or, more recently, the body mass index or BMI. The BMI is defined as the ratio of weight in kg divided by the height in metres squared. Obesity develops when energy intake is greater than energy expenditure, the excess energy being stored mainly as fat in adipose tissue. Body weight loss and prevention of weight gain can be achieved by reducing energy intake or bioavailability, increasing energy expenditure and /or reducing storage as fat. It has been known for many years that the ingestion of dietary proteins stimulates energy expenditure in the postprandial period immediately after meal ingestion. Certainly, on theoretical grounds, the energy cost of digesting, absorbing, and metabolizing proteins is greater than that of either carbohydrates or fat, and these theoretical values have been supported and confirmed for proteins and carbohydrates in human clinical trials (Tappy L. et al., 1993, Am J Clin Nutr, 57:912-916; Acheson K. et al., 1984, J Clin Invest, 74:1572-1580).

Proteins not only increase energy expenditure, but also decrease energy intake through mechanisms that influence appetite control (Halton TL. et al., 2004, J Am Coll Nutr, 23:373-385; Anderson GH. et al., 2004, J Nutr 134:9745-9795; Lejeune MP. et al., 2005, Br J Nutr, 93:281-289). Thereby, the effect of hyper-aminoacidemia, especially of the branched chain amino acids and more importantly of leucine, is important to enhance energy expenditure that is partly a result of increased protein turnover. Results from many medium-term clinical trials have provided evidence that high-protein diets favour weight loss and reduce biomarkers of related metabolic diseases, at least over periods of several months to several years (Skov A.R. et al. 1999, Int J Obes 23:528-536; Brehm B.J. et al. 2003, J Clin Endocrinol Metab 88:1617-1623; Foster G.D. et al. 2003,N Engl J Med 348:2082-2090; Samaha F.F. et al., 2003, N Engl J Med 348:2074-2081; Due A. et al. 2004, Int J Obes Relat Metab Disord 28:1283-1290).

Further studies have shown that milk proteins are absorbed and digested at different rates, than for example animal and vegetable proteins, and thereby stimulate energy expenditure differently (Boirie Y et al., 1997, Proc Natl Acad Sci USA 94:14930-14935; Mikkelsen PB et al., 2000, Am J Clin Nutr 72:1135-1141). Thus, different proteins appear to have a variety of acute and chronic metabolic effects, thereby affecting postprandial energy expenditure and satiety, and in the medium-term loss of weight, an increase of lean body mass and a decrease of body fat mass.

Whey protein is considered generally as healthy. For example, WO2011/112695 lists a number of health benefits of whey proteins, among them muscle development, control of blood glucose and weight management and satiety.

A recent study (Acheson K et al., 2011, Am J Clin Nutr 93:525-534) investigated the thermic and metabolic responses and the satiating effects of 4 isocaloric test meals on 23 healthy men and women. Three of the meals provided 50% of the energy as whey, casein or soy proteins, respectively, and one meal was an iso-energetic high-carbohydrate meal as control. The results indicated that the energy expenditure as well as the thermic effect of the protein meals was higher than those of the carbohydrate rich meal. Further, among the protein rich meals, the whey protein meal showed the significantly strongest thermic effect and the largest energy expenditure effect on the tested subjects. Cumulative fat oxidation was also largest after the whey protein meal in comparison to the other 3 provided meals.

There is still a persisting need in the food industry to find nutritional solutions for overweight subjects or subjects at risk of becoming overweight for better managing their body weight, e.g. through increasing satiety, postprandial energy expenditure, enhancing lean body mass and/or reducing body fat mass.

The object of the present invention is to improve the state of the art and to provide a new solution for the prevention and/or treatment of overweight and obesity in a subject of such need.

The object of the present invention is achieved by the subject matter of the independent claims. The dependent claims further develop the idea of the present invention.

Accordingly, the present invention provides whey protein micelles for use in the prevention or treatment of overweight and/or obesity in a subject, wherein the whey protein micelles are administered to said subject in combination with a meal, wherein the meal comprises whey protein hydrolysates, native or hydrolysed milk proteins, free amino acids, or a combination thereof.

In a second aspect, the present invention relates to a non-therapeutic use of whey protein micelles to increase satiety and/or postprandial energy expenditure in a subject, wherein the whey protein micelles are administered to said subject in combination with a meal, wherein the meal comprises whey protein hydrolysates, native or hydrolysed milk proteins, free amino acids, or a combination thereof.

A still further aspect of the present invention is a food composition comprising whey protein micelles, wherein the food composition is to be administered to an overweight or obese subject, or to a subject at risk for becoming overweight or obese.

"Whey protein micelles" (WPM) are defined herein as described in EP1839492A1 and as further characterized in Schmitt C et al. (2010, Soft Matter 6:4876-4884), where they are referred to as whey protein microgels (WPM). Particularly, the "whey protein micelles" are the micelles comprised in the whey protein micelles concentrate obtainable by the process as disclosed in EP1839492A1. Therein, the process for the production of whey protein micelles concentrate comprises the steps of: a) adjusting the pH of a whey protein aqueous solution to a value between 3.0 and 8.0; b) subjecting the aqueous solution to a temperature between 80 and 98°C; and c) concentrating the dispersion obtained in step b). Thereby, the micelles produced have an extremely sharp size distribution, such that more than 80% of the micelles produced have a size smaller than 1 micron in diameter and preferably are between 100 nm and 900 nm in size. The "whey protein micelles" can be in liquid concentrate or in powder form. Importantly, the basic micelle structure of the whey proteins is conserved, in the concentrate, the powder and reconstituted from the powder for example in water. The "whey protein micelles" are physically stable in dispersion, as powder as well as during spray-drying or freeze-drying.

"Overweight" is defined for an adult human as having a BMI between 25 and 30. Thereby, BMI (body mass index) means the ratio of weight in kg divided by the height in metres, squared.

"Obesity" is a condition in which the natural energy reserve, stored in the fatty tissue of animals, in particular humans and other mammals, is increased to a point where it is associated with certain health conditions or increased mortality. "Obese" is defined for a human as having a BMI greater than 30.

It has been surprisingly found by the inventors that whey protein micelles consumed as part of a meal induce the same high plasma aminoacidemia as an iso-caloric and iso-nitrogenous control meal with whey protein isolates (WPI), but significantly delayed postprandial amino acid appearance in the blood by about 30 min with respect to that of the control meal. Consequently, surprisingly, the micelle structure generates an improved health benefit as compared to normal whey protein. To the inventors best knowledge this has never been reported. The peak amino acid concentration (i.e. Cmax) after the whey protein micelles meal was the same as after the WPI meal, and significantly higher than the maximum concentrations reached after an iso-caloric and iso-nitrogenous milk protein or milk casein meal. The results of the clinical study are presented in the Example section. Hence, the inventors have found a protein composition which when consumed as part of a regular meal induces a delayed but high maximal aminoacidemia in a subject. This hyper-aminoacidemia for a prolonged postprandial period of time is most favorable for maximally stimulating and increasing the postprandial energy expenditure and energy partitioning of the subject to improve body mass composition and control body weight. The resulting increase in thermogenesis will also enhance satiety.

"Hyper-aminoacidemia" is an excess of amino acids in the bloodstream, the amino acid pool, which can lead to an increase in both protein synthesis and protein breakdown through protein oxidation, with an overall positive nitrogen balance. Thereby, the positive nitrogen balance indicates more construction of lean tissue than destruction, leading overall to an increase in lean body mass and hence reduction of body fat mass.

Although not wishing to be bound by theory, the inventors think that whey protein micelles as part of a meal seem to induce a delayed gastric emptying or to be more slowly digested as compared to native whey proteins such as WPI. Thereby, whey protein micelles deliver the amino acids more slowly into the peripheral blood circulation.
Figure 1: Plasma concentrations of essential amino acids 3 h after the ingestion of meal replacements comprising whey protein isolate, whey protein micelles or micellar casein.
Figure 2: Plasma concentrations of leucine 3 h after the ingestion of meal replacements comprising whey protein isolate, whey protein micelles or micellar casein.
Figure 3: Plasma concentrations of essential amino acids 3 h after the ingestion of meal replacements comprising each one of the 7 different proteins.

The present invention pertains to whey protein micelles for use in the prevention or treatment of overweight and/or obesity in a subject, wherein the whey protein micelles are administered to said subject in combination with a meal.

Most meals comprise proteins from a milk, plant and/or animal source and hence upon consumption lead to a postprandial aminoacidemia, i.e. an elevated concentration of amino acids in the plasma of the consumer. It is now of an advantage, to combine the administration of whey protein micelles in combination with such a meal. Thereby, the postprandial plasma amino acid peak resulting from the proteins present in the meal adds up to the postprandial amino acid peak resulting from the whey protein micelles which are delayed by ca. 30 min in respect to the first amino acid peak. Thereby, the overall resulting high aminoacidemia is extended and prolonged in time. This in return is most favorable for maximally stimulating and increasing the postprandial energy expenditure and energy partitioning to improve body mass composition and control body weight of a subject.

The meal comprises whey protein isolates, native or hydrolyzed milk proteins, free amino acids, or a combination thereof. As known from earlier studies, a whey protein meal exhibits a significantly stronger thermic and larger energy expenditure effect on subjects than for example a plant protein meal. Therefore, advantageously, the whey protein micelles are combined with a meal comprising whey proteins in the form of WPI or milk. Advantageously, the meal can be even further supplemented with free amino acids in combination with the whey or milk proteins to optimally induce a strong plasma aminoacidemia effect upon consumption of said meal.

In a further embodiment, the whey protein micelles are provided as part of the meal in the form of a beverage, nutritional composition, bar, flakes or as pellets. Those forms of food product applications are ideal for incorporating whey protein micelles in a sufficient quantity for providing the desired effect and still be acceptable by a consumer in view of the organoleptic aspect.

In a preferred embodiment, the whey protein micelles are administered to a subject in a daily dose of at least 20 g dry weight, preferably of at least 30 g dry weight. Those doses should assure a sufficient daily quantity for providing the desired effect to a subject in at least a mid-term period.

The whey protein micelles for use according to the invention pertain to a subject which is a child or an adult human being. Alternatively, the subject is an animal, preferably a cat or a dog. Prevalence of obesity is mostly observed in adult humans. However, more and more children are affected as well and/or are already at risk of becoming overweight or obese later in life. Hence, advantageously, prevention and/or treatment of overweight is started already in young age. Alternatively, and similarly as observed with humans, obesity is more and more widespread among animals, particularly with animals kept as pet animals. Hence, the invention preferably also pertains to cats and dogs.

A further aspect of the present invention is the non-therapeutic use of whey protein micelles to increase satiety and/or postprandial energy expenditure in a subject, and particularly to enhance lean body mass and/or decrease body fat mass, as defined by the claims.

A still further aspect related to the invention is the non-therapeutic use of whey protein micelles to help maintaining a healthy body composition after weight loss.

It is an advantage of the present invention that whey protein micelles can also be administered to healthy subjects which may be at risk of developing overweight. In fact, whey protein micelles or a food composition comprising whey protein micelles as disclosed herein provide healthy humans and animals with increased satiety and/or increased energy expenditure after consumption of said whey protein micelles. The effect is due to the herein disclosed sustained and prolonged hyper-aminoacidemia postprandial effect. Further, this effect is most favorable for improving body mass composition, such as enhancing lean body mass, and controlling body weight by decreasing for example body fat mass. It is a legitimate desire also for healthy subjects to stay healthy and slim.

A further aspect of the present invention is a food composition comprising whey protein micelles, wherein the food composition is to be administered to an overweight or obese subject, or to a subject at risk for becoming overweight or obese; and wherein the food composition comprises the whey protein micelles in an amount of at least 15 wt%, preferably of at least 20 wt% of the total dry weight of such food composition.

In a preferred embodiment, the food composition comprises 15-50 wt% proteins, 10-15 wt% lipids, 25-50 wt% carbohydrates and 5-10 wt% fibers of total dry weight.

A still further aspect of the present invention is a pet food composition comprising whey protein micelles.

Those skilled in the art will understand that they can freely combine all features of the present invention disclosed herein. In particular, features described for the therapeutic uses of the whey protein micelles may be combined with those of the non-therapeutic uses, and vice versa. Idem, the features of the therapeutic and non-therapeutic uses may be combined with the features to the food composition, and vice versa. Further, features described for different embodiments of the present invention may be combined. Further advantages and features of the present invention are apparent from the figures and examples.

### Example

A randomized double-blind 7-arm crossover study was performed in twenty-three healthy men in the following way. A test meal replacement was ingested at lunch time on 7 separate occasions separated each by a wash-out period of one week. The meal replacements were iso-caloric and iso-nitrogenous. They were composed of the tested protein (30g, 7.2% w/w), lipids (11.7g, 2.8% w/w), carbohydrates (42.7g, 10.2% w/w) and fibers (6.3g, 1.5% w/w). The tested proteins were: (1) whey protein isolate (WPI); (2) whey protein micelles (WPM); (3) extensively hydrolyzed whey protein (EHWP); (4) micellar casein (ICP); (5) extensively hydrolyzed casein protein (EHCP); (6) total milk proteins (TMP); and (7) extensively hydrolyzed milk proteins (EHMP). The meal replacements were completed with water to 430mL and contained 388kcal per serving.

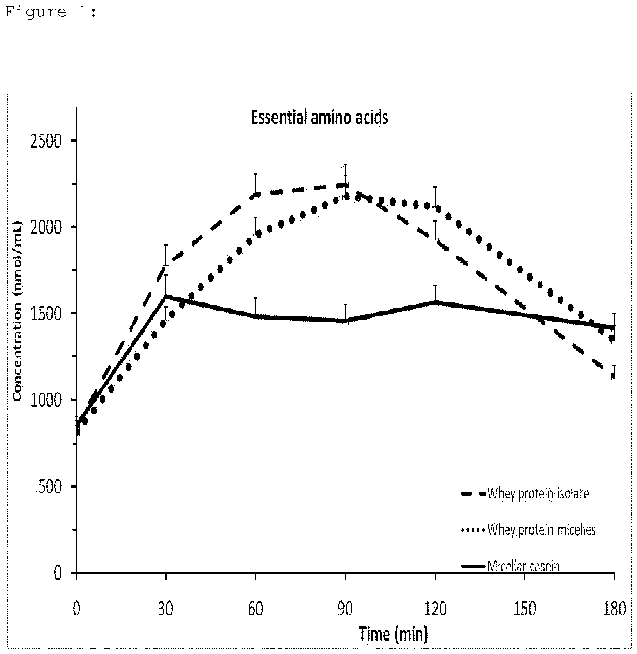

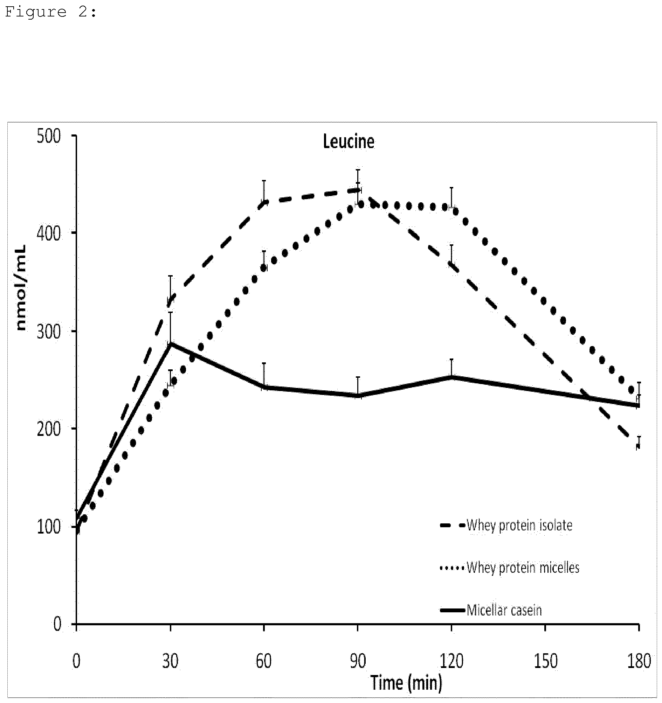

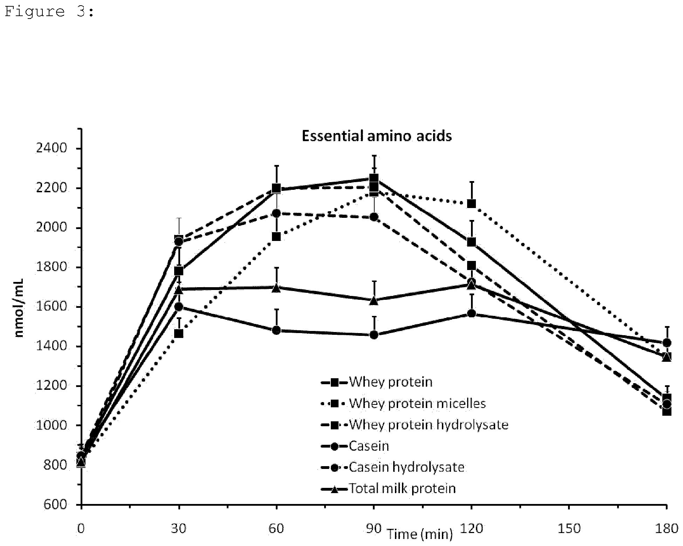

Arterialized venous blood samples were taken, via a catheter inserted into a wrist vein of the volunteers, before and for 3h after consuming the test meal replacement. Plasma samples were used to analyze amino acids by gas chromatography and mass spectrometry. The results are shown in Figures 1 to 3.

Firstly, the results confirmed that intact whey protein induces a higher aminoacidemia than micellar casein. Secondly, it was found that the peaks of the postprandial plasma amino acid concentrations after consumption of the WPI and WPM test meal replacements, although similar in extent and height, were delayed by approximately 30 min, i.e. occurring at 120 min rather than at 90 min. This allowed maintenance of an elevated concentration of plasma amino acids for a prolonged period of time after the ingestion of the whey protein micelles (Figures 1 to 3: small dotted lines).

## Claims

1. Whey protein micelles for use in the prevention or treatment of overweight and/or obesity in a subject, wherein the whey protein micelles are administered to said subject in combination with a meal, wherein the meal comprises whey protein isolates, native or hydrolyzed milk proteins, free amino acids, or a combination thereof.

2. The whey protein micelles for use according to claim 1, wherein the whey protein micelles are provided as part of the meal in the form of a beverage, nutritional composition, bar, flakes or as pellets.

3. The whey protein micelles for use according to one of the preceding claims, wherein the whey protein micelles are administered to said subject in a daily dose of at least 20 g dry weight, preferably of at least 30 g dry weight.

4. The whey protein micelles for use according to one of the preceding claims, wherein the subject is a child or an adult human being.

5. The whey protein micelles for use according to one of the claims 1-4, wherein the subject is an animal, preferably a cat or a dog.

6. Non-therapeutic use of whey protein micelles to increase satiety and/or postprandial energy expenditure in a subject, wherein the whey protein micelles are administered to said subject in combination with a meal, wherein the meal comprises whey protein isolates, native or hydrolyzed milk proteins, free amino acids, or a combination thereof.

7. The non-therapeutic use according to claim 6, to enhance lean body mass and/or decrease body fat mass.

## Patentansprüche

1. Molkenproteinmizellen zur Verwendung in der Prävention oder Behandlung von Übergewicht und/oder Fettleibigkeit bei einem Probanden, wobei die Molkenproteinmizellen dem Probanden in Kombination mit einer Mahlzeit verabreicht werden, wobei die Mahlzeit Molkenproteinisolate, native oder hydrolysierte Milchproteine, freie Aminosäuren oder eine Kombination davon umfasst.

2. Molkenproteinmizellen zur Verwendung nach Anspruch 1, wobei die Molkenproteinmizellen als Teil der Mahlzeit in Form eines Getränks, einer Nährstoffzusammensetzung, eines Riegels, in Form von Flocken oder als Pellets bereitgestellt werden.

3. Molkenproteinmizellen zur Verwendung nach einem der vorstehenden Ansprüche, wobei die Molkenproteinmizellen dem Probanden in einer täglichen Dosis von mindestens 20 g Trockengewicht, vorzugsweise von mindestens 30 g Trockengewicht verabreicht werden.

4. Molkenproteinmizellen zur Verwendung nach einem der vorstehenden Ansprüche, wobei es sich bei dem Probanden um ein Kind oder um einen Erwachsenen handelt.

5. Molkenproteinmizellen zur Verwendung nach einem der Ansprüche 1 bis 4, wobei es sich bei dem Probanden um ein Tier, vorzugsweise um eine Katze oder einen Hund, handelt.

6. Nicht-therapeutische Verwendung von Molkenproteinmizellen, um das Sättigungsgefühl und/oder den postprandialen Energieverbrauch bei einem Probanden zu erhöhen, wobei die Molkenproteinmizellen dem Probanden in Kombination mit einer Mahlzeit verabreicht werden, wobei die Mahlzeit Molkenproteinisolate, native oder hydrolysierte Milchproteine, freie Aminosäuren oder eine Kombination davon umfasst.

7. Nicht-therapeutische Verwendung nach Anspruch 6, um die Körpermagermasse zu erhöhen und/oder die Körperfettmasse zu verringern.

## Revendications

1. Micelles de protéines de lactosérum destinées à être utilisées dans la prévention ou le traitement du surpoids et/ou de l'obésité chez un sujet, où les micelles de protéines de lactosérum sont administrées audit sujet en combinaison avec un repas, dans lesquelles le repas comprend des isolats de protéines de lactosérum, des protéines de lait natives ou hydrolysées, des acides aminés libres, ou une combinaison de ceux-ci.

2. Micelles de protéines de lactosérum utilisable selon la revendication 1, où les micelles de protéines de lactosérum sont fournies dans le cadre du repas sous la forme d'une boisson, d'une composition nutritionnelle, d'une barre, de flocons ou en tant que granulés.

3. Micelles de protéines de lactosérum utilisables selon l'une des revendications précédentes, où les micelles de protéines de lactosérum sont administrées audit sujet en une dose journalière d'au moins 20 g de poids sec, de préférence d'au moins 30 g de poids sec.

4. Micelles de protéines de lactosérum pour une utilisation selon l'une des revendications précédentes, dans lesquelles le sujet est un enfant ou un être humain adulte.

5. Micelles de protéines de lactosérum utilisables selon l'une des revendications 1 à 4, dans lesquelles le sujet est un animal, de préférence un chat ou un chien.

6. Utilisation non thérapeutique de micelles de protéines de lactosérum pour augmenter la satiété et/ou la dépense énergétique postprandiale chez un sujet, dans laquelle les micelles de protéines de lactosérum sont administrées audit sujet en combinaison avec un repas, dans laquelle le repas comprend des isolats de protéines de lactosérum, des protéines de lait natives ou hydrolysées, des acides aminés libres, ou une combinaison de ceux-ci.

7. Utilisation non thérapeutique selon la revendication 6, pour améliorer la masse corporelle maigre et/ou diminuer la masse graisseuse corporelle.
